# EUROPEAN PATENT SPECIFICATION

(11) **EP 2 081 499 B1**
(45) Date of publication and mention of the grant of the patent: **04.05.2016**
(21) Application number: 07863764.2
(22) Date of filing: 01.11.2007
(51) Int. Cl.: A23L 2/38, A23L 2/52, A61B 5/00, A61B 5/145, A61B 10/00, G01N 33/50, G01N 33/68, G01N 33/84

(54) **LIMITING MUSCLE CRAMPS**
BEGRENZUNG VON MUSKELKRÄMPFEN
LIMITATION DES CRAMPES MUSCULAIRES

(30) Priority: 01.11.2006 US 591209
(43) Date of publication of application: 29.07.2009
(60) Divisional application: 16157558.4
(73) Proprietor: Stokely-Van Camp, Inc., Chicago, IL 60661 (US)
(72) Inventor: MURRAY, Robert, Fox River Grove, IL 60021 (US); STOFAN, John R., Deer Park, IL 60010 (US); ZACHWIEJA, Jeffrey J., Carey, IL 60013 (US); HORSWILL, Craig A., Barrington, IL 60010 (US); RAPKIN, Myron C., Indianapolis, IN 46220 (US); WEBER, Wayne W., Fortville, IN 46040 (US)
(74) Representative: Duxbury, Stephen
(86) International application number: PCT/US2007/083305
(87) International publication number: WO 2008/057922

(56) References cited:
- EP-A- 0 475 045
- WO-A-2006/023985
- GB-A- 1 252 781
- US-A- 3 552 929
- US-A- 5 361 758
- US-A1- 2003 201 192
- US-A1- 2005 100 637
- US-A1- 2006 121 548
- US-A1- 2006 253 011
- MAUGHAN RONALD J ET AL: "Fluid and electrolyte intake and loss in elite soccer players during training." INTERNATIONAL JOURNAL OF SPORT NUTRITION AND EXERCISE METABOLISM JUN 2004, vol. 14, no. 3, June 2004 (2004-06), pages 333-346, XP008090402 ISSN: 1526-484X

## Description

### FIELD OF THE INVENTION

The present invention relates generally to muscle cramps and more particularly to devices, systems and methods relating to monitoring body fluid components and parameters, the reduction of cramping, and related aspects.

### BACKGROUND

Muscles cramps, the involuntary and forceful contraction of muscles, are painful and may last for a prolonged period of time. During physical activities in hot weather conditions, cramps appear to occur more often. Such types of cramps are commonly referred to as heat cramps. Due to the pain and lack of movement of the affected limb, the physical activity is interrupted at least until the cramping stops. Even then, re-exerting the muscle may cause the cramping to reoccur. As such, muscle cramping may impact an athlete for a prolonged period of time. In the case of an athletic competition, its outcome may be adversely impacted due to withdrawal of or inability to perform by the affected athlete.

Reliable methods and systems of monitoring salt depletion would provide more accurate and timely indications of muscle cramping. Further methods and systems may be implemented to reduce or avoid muscle cramps from occurring.

US 3552929 discloses a device for detecting halide ion concentration in fluids. WO 2006/023985 discloses a system and method for modifying a fluid for oral administration. US 2006/0121548 discloses a system and method for measuring sodium concentration in saliva. EP 0475045 discloses a method and device for the assay of ions.

US 5361758 discloses a method and device for measuring concentration levels of blood constituents non-invasively.

US 2003/0201192 describes a disposable, self-administered electrolyte test. US 2005/0100637 is directed towards a carbohydrate and electrolyte replacement composition.

### SUMMARY

The present invention is directed towards the product for reducing muscle cramps of independent Claim 1, the use of this product for reducing muscle cramps during physical activity, and the use of the consumable composition of this product for reducing muscle cramps during physical activity. The dependent claims specify preferred but optional features. The product includes a kit for detecting changes in body fluid components and/or parameters of bodily fluid during physical exertion. Such a kit includes tester and may include dosage information for consuming a beverage based upon results of a test with the tester. In such an embodiment, the tester includes a test portion. The test portion, when exposed to body fluid, reacts to produce a result indicating the degree of ionic depletion from the test subject. The tester may be configured to measure components in body fluid, such as salt or ionic loss, to estimate adequate replacement of such components during, for example, physical activity. In an exemplary embodiment, a perspiration sample is tested to indicate ionic loss, thereby monitoring the amount of ions depleted from the body. Such sampling may occur during physical exertion, thus providing more accurate results. The sample may then be analyzed to provide a result which indicates a degree of ionic depletion. Based on the degree of ionic depletion, dosage information may be provided to replenish the body with sufficient amounts of such things as salt to reduce the likelihood of cramping.

Yet in one or more additional embodiments, other types of body fluids collected may be used, instead of or in addition to a perspiration sample, to test ionic content or other parameters, such as for example, pH and/or specific gravity. Yet in still further embodiments, the results from such samples may be used for one or more other purposes. In an embodiment, a sample collection unit may be provided as part of a kit. In one embodiment, the sample collection unit comprises a pouch with first and second surfaces and a space there between. In such an embodiment, an opening is located at or near a first end of the pouch. The opening may facilitate collection of a sample of body fluid when the collection unit is dermally mounted onto the skin of a test subject. Other embodiments may include an absorbent layer passing through said opening to further aid the collection of body fluid.

The invention is directed to a product for reducing muscle cramps. In an exemplary embodiment, the product comprises a primary product which contains salt of one or more elements. A kit may be provided with the primary product. The kit includes a sample collection unit having a pouch with first and second surfaces and an opening located at or near a first end thereof. The opening may facilitate collection of a sample of body fluid when the collection unit is dermally mounted onto the skin of a test subject. A tester is also provided with the kit. The tester includes a test portion which, when exposed to the sample after it has been collected, reacts with the sample to produce a result indicating the degree of ionic depletion from the test subject. In various embodiments, the tester may also be configured to measure the ionic concentration of sodium, chloride, magnesium, potassium and combinations thereof. The tester may also configured to measure the concentration of components selected from the group consisting of: proteins, urea, ketones, lactate, and combinations thereof. Still in yet further embodiments, the tester may be configured to measure parameters selected from the group consisting of: specific gravity, pH, and combinations thereof.

These and other objects, along with advantages and features of the inventive embodiments herein disclosed, will become apparent through reference to the following description and the accompanying drawings. Furthermore, it is to be understood that the features of the various embodiments described herein are not mutually exclusive and may exist in various combinations and permutations.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention. In the following description, various embodiments of the invention are described with reference to the following drawings, in which:
Figs. 1 a-b show different views of a exemplary sample collection unit in accordance with one embodiment of the invention;
Figs. 2a-b show different views of a sample collection unit that does not form part of the claimed invention;
Fig. 3 shows an exemplary tester in accordance with one embodiment of the invention;
Fig. 4 shows an exemplary test chart in accordance with one embodiment of the invention;
Fig. 5 shows an exemplary product package including a test kit in accordance with one embodiment of the invention; and
Fig. 6 shows an exemplary process for limiting muscle cramps.

### DETAILED DESCRIPTION

As used herein, the terms "limit" and "limiting" refer to the reduction of muscle cramping. In some embodiments, muscle cramping may be substantially reduced to a level where it may be considered non-existent. According to one or more embodiments described in more detail below, muscle cramps may be limited by monitoring ionic or salt loss during, for example, physical activity. To monitor salt loss, a perspiration sample is obtained and tested. Testing salt content from other types of body fluids collected or using the results for other purposes, such as urine, is also contemplated within the scope of the invention. In various embodiments, the tester may also be configured to measure the ionic concentration of sodium, chloride, magnesium, potassium and combinations thereof. The tester may also configured to measure the concentration of components selected from the group consisting of: proteins, urea, ketones, lactate, and combinations thereof. Still in yet further embodiments, the tester may be configured to measure parameters selected from the group consisting of: specific gravity, pH, and combinations thereof. As used herein, the term "tester" is utilized in the singular form, however, as with other terms used throughout this disclosure, the usage of "tester" is not limited on only a single tester. Rather, one or more testers, each configured to measure one or more components and/or parameters from body fluid, are within the scope of the invention.

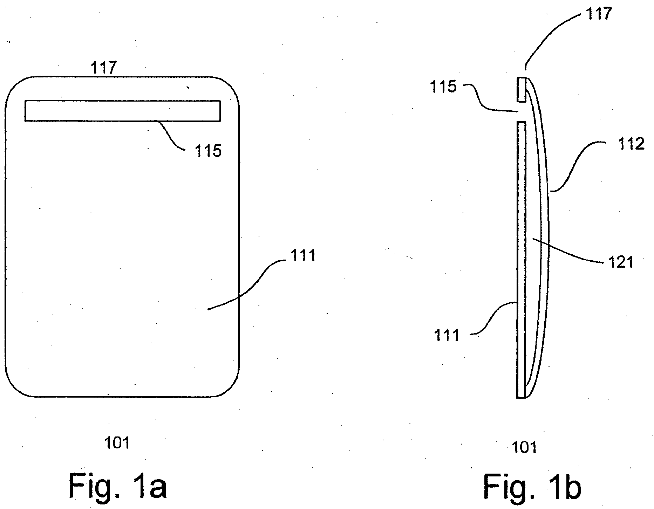

Figs. 1 a-b show planar and side views of an exemplary collection unit 101 used to collect a sample for testing in accordance with an embodiment of the invention. The collection unit collects and stores bodily fluids, such as perspiration ("sample"). As shown, the collection unit 101 comprises a pouch having an internal space 121 between first and second sides 111 and 112. In one embodiment, an opening 115 is provided at or near a first end 117 of the pouch to facilitate collection and storage of the sample in the space. As will be appreciated by those skilled in the art upon review of this disclosure, the pouch may have various shapes, for example, rectangular in shape with rounded corners, as shown in the Figures. Providing a pouch with other geometric shapes is also useful and may depend on a myriad of factors, including but not limited to: manufacturing restrictions, ergonomics, costs, design considerations, and the amount of sample to be collected.

The size of the pouch should be sufficient to hold at least a desired amount of the sample for testing. In certain embodiments, the amount of sample to be tested is about 10ml to about 30 ml., yet in other embodiments, the amount of the sample may be '/2 oz. or 15 ml. and may range from as low as 5 ml. with no specific upper range. In one embodiment, the dimensions of the pouch are, for example, about 6 cm. by about 9 1/2 cm. Other dimensions may also be useful, and any size pouch may be used that may be attached to the body.

In an exemplary embodiment, the pouch is formed by attaching or bonding the edges of first and second layers together leaving the interior portion un-bonded to create the space. Other techniques for forming the pouch may also be useful. Such techniques, for example, may include injection or blow molding; however, there are any number of other methods of creating the collection unit 101. The pouch should be formed from a material which may adequately store or hold the collected sample. In one example, material for forming the pouch comprises Tegaderm® trademarked product, commercially available from 3M, Minneapolis, Minnesota or Pharmcheck™ trademarked product, commercially available from PharmChem, Inc., Fort Worth, TX. Other materials may include any type of material that may be flexible and waterproof.

The opening 115 may be provided by not bonding the edges of the layers at the first end 117 of the pouch. Alternatively, the opening may provided near the first end of the first layer. The opening should be sufficiently large to enable collection of the sample. In one embodiment, the opening extends to at least about half the width of the pouch. In yet other embodiments, the opening extends to at least about 3/4 the width of the pouch. In still yet further embodiments, the opening extends to about 90 percent of the width of the pouch.

To facilitate the flow of the sample into the pouch, an absorbent layer (not shown) may be provided at the opening. Various types of materials which enhance the collection of the sample may be used to form the absorbent layer. Such materials may include, for example, fibrous materials and/or hydrophilic membranes. For example, the absorbent layer may be formed from hydrophilic polymer fibers such as polycarbonate, cellulose acetate, nylon or combinations thereof. Other types of materials, such as natural fibers which include paper, cloth cellulose or synthetic fibers may also be used. However, it must be verified that such material does not affect the salt content of the collected liquid. The absorbent layer, in one embodiment, includes a first portion that extends outside of the opening and a second portion disposed inside the space. Other configurations of the absorbent layer may also be useful.

In certain examples an adhesive may be used to fix the absorbent layer to the pouch. In one such embodiment, the adhesive may also be provided to fix the portion of the absorbent layer which extends out of the opening to the skin. Various types of adhesives may be used, including any adhesive that is used in medical or personal care products. Suggested adhesives used would not interact with the sample, causing contamination and which may produce inaccurate test results. In one embodiment, an adhesive is disposed on the outer surface of the first layer of the pouch. The adhesive temporarily attaches the collection unit 101 to the skin of the user. The use of an adhesive allows the collection unit 101 to be conveniently attached to various parts of the body. For example, the collection unit 101 may be attached to a part of the body which facilitates collection of the sample as well as being easily accessed by the user.

Various types of adhesives may be used, where suggested adhesives would be inert with the sample being collected. Additionally, in certain embodiments, the adhesive used would not cause skin irritation to the user and may optionally include agents to reduce irritation. Other types of adhesives, such as those used in topical patches or adhesive tapes may be useful. Yet in other embodiments, the collection unit 101 may be physically restrained to the body, such as through the use of straps, clips, elastic bands, or any other suitable means. The placement of the collection unit 101 may depend on a myriad of factors, including, but not limited to: the size, type and amount of desired sample, comfort, and other factors. The inventors have determined that the chest or waist of the body are suitable locations to place the collection unit 101.

When attached to the body, the collection unit 101 may be oriented so that the opening 115 is located at the top or substantially at the top. In such embodiments, gravity may assist the flow downwards through the opening 115 and into the pouch. In certain embodiments, the absorbent layer, if present, further attracts the sample through the opening 115 and into the pouch.

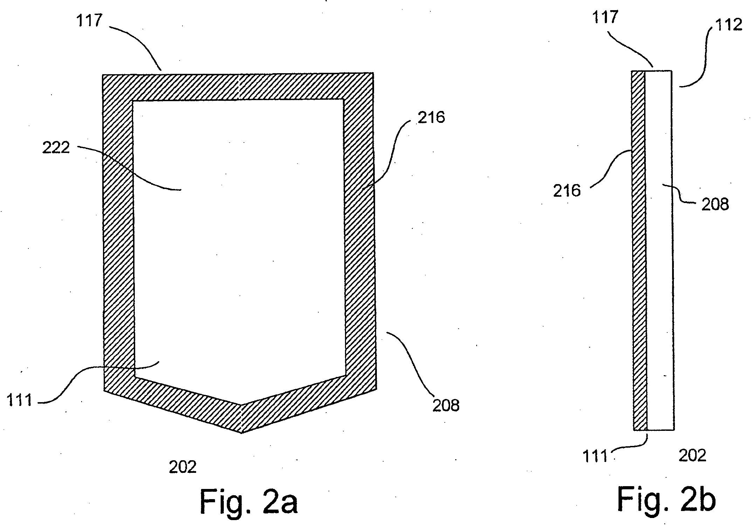

Figs. 2a-b show planar and side views of an exemplary collection unit 202 that does not form part of the claimed invention. As shown, collection unit 202 comprises a patch having a layer 208 with first and second sides 111 and 112. The pouch may have various shapes. The pouch, for example, may have a pentagonal shape, with three sides being arranged in 90° angles to each other while the other two sides being a V, forming a shape similar to that of a pocket. Providing a pouch with other geometric shapes is also useful. The layer should be formed from a material which may adequately store or hold the collected sample. For example, in select embodiments, the layer may be formed from the products described above.

An adhesive 216 may be disposed on a first surface of the patch. In one example, the adhesive is disposed at about the circumference of the patch, leaving the internal portion 222 of the patch devoid of the adhesive. The adhesive attaches the collection unit onto the skin of the user. The use of an adhesive 216 allows the collection unit 202 to be conveniently attached to various parts of the body. In certain embodiments, the patch is oriented such that the V, created by adherence of the layer to the skin of a user, is located toward the bottom of the patch, closer to the ground. However, the patch may also be attached to the skin with other orientations.

When attached, the patch forms a cavity or space between it and the skin, substantially sealed by the adhesive. The cavity corresponds to the inner portion of the patch which does not contain adhesive. Sample collected is then stored in this cavity until ready for testing. Testing may be conducted by lifting a top edge 117 of the patch is lifted off the skin to form an opening though which a tester may be dipped to access the sample. In an alternative embodiment, the opening may be provided by not providing adhesive at least at a portion of the top edge 117. The portion should be sufficiently large to create an opening though which a tester may pass.

In certain embodiments, a tester or test strip may be used by inserting it into the pouch after the sample has been collected. The tester may be made to turn colors depending on the content of the sample. The tester may then be removed from the pouch and compared, for example, to a color strip which informs the user of the ionic or salt concentration in the sample; wherein each color identifies a different concentration level. In the alternative, a test strip may be located in the pouch, and the color strip may be either outside - so the comparison may be made by comparing the test strip color through a see-through pouch - or both the test strip and the color strip may be with the pouch with both visible through the see-through pouch.

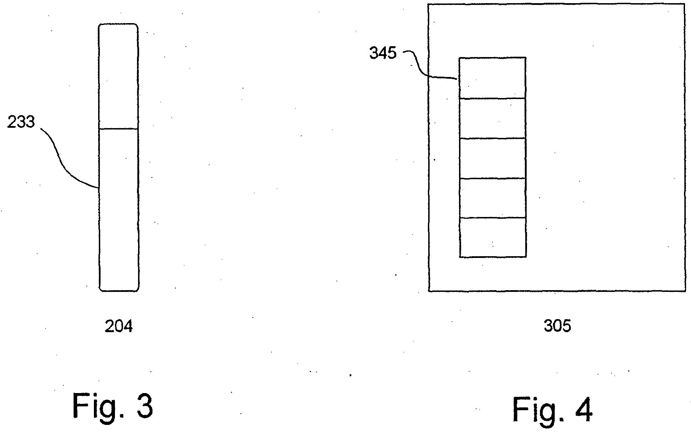

Fig. 3 shows an exemplary tester 204 in accordance with one embodiment of the invention. The tester 204, for example, comprises a test strip which includes a test portion 233. In an exemplary embodiment, the tester may be made from a material, such as filter paper, which is able to absorb the sample. To test the sample, the tester may be dipped into the pouch via the opening such that the test portion 233 comes into contact with the sample. The test portion 233 of the tester 204 produces a result based on the content of a target component in the sample. The tester 204, in one embodiment, produces a result based on the salt content of a perspiration sample, indicating the degree of salt depletion. Testing for other types of target components may also be useful.

In one embodiment, the tester 204 utilizes a colorimetric technique to determine the content of a target component in the sample, such as the ionic load of the sweat. In such an embodiment, a reagent which reacts with a salt may be disposed in the test portion. In one embodiment, the reagent reacts with chloride. Other elements that may be measured include sodium, the specific gravity, pH, proteins, urea, ketones, lactate, magnesium or potassium. In certain testing procedures, the reaction causes the test portion to develop a specific color depending on the concentration of the target component. The result from the tester 204 indicates the degree of ionic depletion.

In another example, the test portion may include a plurality of segments with respective reagents or reagent compositions. A segment which corresponds to the salt concentration in the sample reacts and develops accordingly, for example, a specified color. Other types of measurement may include measuring an electrical current through the sweat as a measurement of the ionic load of the sweat.

Fig. 4 shows an exemplary test chart 305 in accordance with one embodiment of the invention. The test chart 305 includes information regarding the test results. In one embodiment, the test chart 305 includes information which indicates the degree of salt depletion based on the information provided by a tester, such as tester 204. In one embodiment, a color scale 345 is provided in the test chart. Other types of scales may be useful, depending on the application. Various colors corresponding to the colors that may be generated by the tester are included in the color scale. The scale may be arranged with discrete colors or a continuous color spectrum which includes color transitions or gradients from one color to the other. The continuous color spectrum may be parsed into segments corresponding to a degree of salt depletion.

The ionic load of the sweat is one factor in determining if ionic replacement is required. Other factors, may include, for example, the quantity of sweat secreted from the body. Thus, in certain embodiments, a total ionic depletion may be measured through measuring the loss of weight of the person through sweat as well as the level of ionic concentration in the sweat. With that total depletion, the quantity of ionic replacement required may be calculated. However, the calculation need not be so exact in other embodiments. For example, the user may estimate total sweat loss by determining whether the level of sweat has been low, medium or high and by knowing the user's weight. So, for example, tables may be provided that estimate total sweat based on the level of sweat (i.e. low, medium or high) and the person's weight. This information may then be combined with the ionic concentration in the sweat to determine the ionic replacement required.

The test chart may also include dosage information, for example, about an amount of salt and liquid to consume based on the degree of salt depletion. The dosage information may be general or related to one or more specific products available in the market for oral rehydration purposes, for example, sports or energy beverages such as Propel® or Gatorade®, commercially available from from PepsiCo, Purchase, NY.

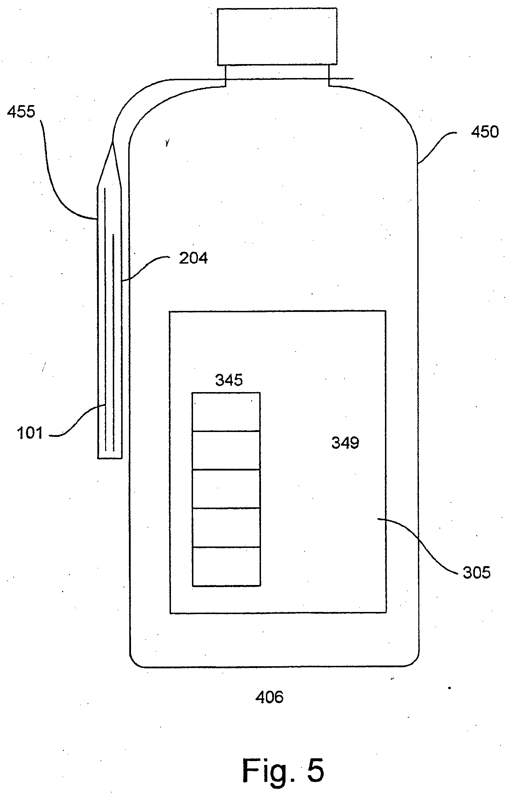

In accordance with one embodiment of the invention, a kit comprising a collection unit, a tester and, optionally, a test chart is provided to monitor the degree of salt loss. Fig. 5 shows an exemplary product package 406 in accordance with one embodiment of the invention. The product package comprises a primary product 450 and a kit for monitoring salt loss. The primary product (PP), in one embodiment, comprises a salt containing oral rehydration beverage. Additionally, non-beverage type products may be formulated in further embodiments based on the guidance given herein and are within the scope of this disclosure. Such other product forms may include, but are not limited to, sports or performance bars, sports or performance gels, chewing gum, sublingual strips, gummy-based products, and confectionery type products. These are merely exemplary of other product forms that can be designed for reducing or avoiding muscle cramping based on the teachings herein. Other product forms will be within the skill of a product formulator when considered together with the teachings provided herein.

Regarding embodiments utilizing a beverage, the beverage may be any type of beverage, including sports or energy beverages. For example, the beverage may comprise Gatorade® or Propel®, commercially available from PepsiCo, Purchase, NY. As shown, the exemplary PP of Fig. 5 is packaged in a bottle-type container, however, the beverage may also be packaged in other types of containers. Providing a beverage in powder or gel form to be mixed with a liquid for consumption may also useful. Packaging the kit with any type of additional products is also useful. For example, mixing utensils may be added to the overall package, condiments may be added, a cup or other vessel may also be added to the package. In one embodiment, the PP may be light and convenient to carry, and usable where water is available. The above embodiments are non-limiting, and other useful combination products for reducing or alleviating muscle soreness are encompassed by this invention.

The exemplary kit shown in Fig. 5, which is provided with the PP, includes a collection unit 101, a tester 204 and a test chart 305 as described above. A portion 349 of the test chart may comprise dosage information. In one embodiment, the dosage information includes at least the amount of PP to be consumed based on the test result may be provided in portion 349 of the test chart. Dosage information of other types of products or general dosage information may also be provided. Additionally, the PP package may include markings corresponding to dosage amounts indicated in the test chart. This may be particularly useful for transparent type of PP packages or where dosage amounts may be less than the total amount of PP contained in the PP package.

In certain embodiments, a kit package 455 may be provided to contain at least some of the kit components. In one embodiment, the kit package includes the collection unit 101 and tester 204 while the test chart 305 is provided with or attached onto the PP package. Other kit packaging arrangements, such as including all components of the kit in the kit package, may also be useful. The kit package may be attached to the PP package. Various techniques may be used for attaching the kit package to the PP package. For example, a bottle net hanger may be provided to attach the test kit to the neck of the PP package. Alternatively, the kit package may be attached to the PP package using an adhesive.

As described, the product package includes one PP and one kit comprising of one each of collection unit, tester and test chart, however, other product package arrangements are also useful. For example, it is not necessary that there is a one to one correspondence with each component of the product package. In some applications, it may be desirable to provide numerous kits per PP or numerous testers within a kit. The packaging arrangement may be tailored for specific applications or needs. Furthermore, the kit or components of the kit may be marketed individually.

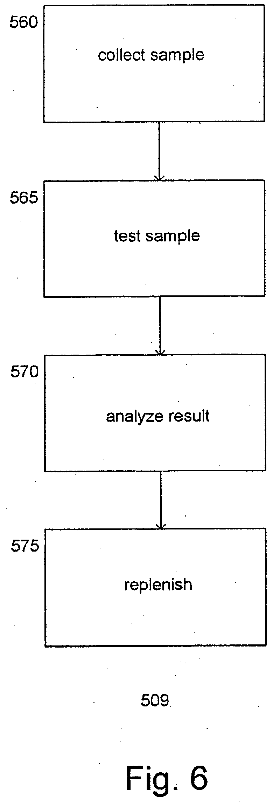

Fig. 6 shows a method for limiting cramps, for example, during physical activities. Over the course of physical activity, sweating occurs. At step 560, a perspiration sample is collected from the user. In one example, the perspiration sample is collected using a collection unit which is attached to the body, such as collection unit 101. Other techniques for collecting perspiration samples may also be useful. The collected sample is tested to determine its salt content at step 565 to indicate the degree of salt depletion. Testing may be conducted using a tester, such as tester 204. The tester, for example, may employ a colorimetric technique. Other test techniques, however, may also be useful.

The test results, at step 570, are analyzed. The analysis involves, for example, using a test chart, such as test chart 305, which contains a scale for determining the degree of salt loss based on the test result. In one example, the test chart 305 comprises a color chart which indicates the degree of salt depletion based on the color of the tester after testing. Based on the degree of salt loss, the appropriate dosage of salt and liquid to consume may be determined. In one example the dosage information may be provided in the test chart 305. This conveniently enables the user to determine the amount of salt needed. The dosage information may be directed to a specific product, such as a rehydration beverage (e.g., Gatorade®). At step 575, the user consumes the recommended amount of rehydration beverage to sufficiently substantially replenish the body with normal level of salt. By monitoring the degree of salt depletion, muscle cramps may be reduced or avoided, particularly with individuals who are early, heavy and salty sweaters.

The invention may be embodied in other specific forms. The foregoing embodiments, therefore, are to be considered in all respects illustrative rather than limiting the invention described herein. Scope of the invention is thus indicated by the appended claims, rather than by the foregoing description.

## Claims

1. A product for reducing or limiting muscle cramps comprising:
a packaging (450) comprising a consumable composition having at least one salt;
a tester (204) including a test portion (233) configured to be exposed to body fluid for use in obtaining a test result indicative of an estimated degree of salt, or ionic, depletion from a test subject; and
a body fluid collection unit (101) configured to be dermally mounted onto the skin of a test subject to collect body fluid during physical exertion, wherein the body fluid collection unit comprises:
a first side (111) substantially secured to a second side (112) at a perimeter, such as to create an internal space (121) between first and second sides (111,112); and
an opening (115) on the first side (111) from the outside of the collection unit (101) to the internal space (121), wherein the opening (115) is configured to allow body fluid to flow within the collection unit (101) through the opening during physical exertion.

2. A product according to claim 1, wherein the collection unit (101) comprises:
an absorbent layer configured to be in substantial contact with the test subject during physical exertion and which layer extends through the opening (115) of the collection unit (101) into the internal space (121), wherein during use, body fluid travels from the test subject through the absorbent layer and into the collection unit (101).

3. A product according to claim 1, wherein the body fluid is perspiration.

4. A product according to claim 1, comprising a table providing an estimate of a quantity of sweat secreted during physical activity, and
comprising dosage information regarding the consumable composition based on the test result for salt replenishment sufficient to substantially limit muscle cramping.

5. A product according to claim 1 or claim 4 further comprising a test chart (305) configured to be compared with the tester (204) after exposure to the body fluid to provide the test result of an estimated degree of salt, or ionic, depletion.

6. The product of claim 5, wherein the test chart (305) comprises at least a portion of the dosage information.

7. The product of claim 5 wherein the at least one salt comprises an electrolyte selected from the group consisting of: sodium, potassium, magnesium, calcium, chloride, and combinations thereof, or,
wherein the consumable composition is selected from the group consisting of: a powder, a gel, a solid, a liquid, and combinations thereof.

8. The product of claim 5 wherein the tester (204) is configured to measure the concentration of components selected from the group consisting of: salt, sodium, chloride, and combinations thereof, or,
wherein the tester (204) is configured to measure the concentration of components selected from the group consisting of: proteins, urea, ketones, lactate, magnesium, potassium and combinations thereof, or,
wherein the tester (204) is configured to measure parameters selected from the group consisting of: specific gravity, pH, and combinations thereof, or,
wherein the product further comprises at least one beverage referenced on dosage information, or,
wherein the product further comprises a rehydration beverage concentrate and dosage information, the dosage information comprising information in preparing a rehydration beverage from the rehydration beverage concentrate.

9. The product according to claim 1 or 2 wherein the tester is configured to be utilized in a colormetric test.

10. Use of a product according to any of the preceding claims for reducing muscle cramps during physical activity.

## Patentansprüche

1. Erzeugnis zum Verringern oder Begrenzen von Muskelkrämpfen, umfassend:
eine Verpackung (450), umfassend eine verzehrbare Zusammensetzung, welche mindestens ein Salz aufweist;
eine Testeinrichtung (204) umfassend einen Testabschnitt (233), der konfiguriert ist, um Körperflüssigkeit ausgesetzt zu werden, zur Verwendung beim Gewinnen eines Testergebnisses, welches ein abgeschätztes Ausmaß an Depletion von Salz oder Ionen von einem Testindividuum anzeigt, und
eine Körperflüssigkeitssammlungseinheit (101), die konfiguriert ist, um dermal auf der Haut eines Testindividuums angebracht zu werden, um Körperflüssigkeit während körperlicher Anstrengung zu sammeln, wobei die Körperflüssigkeitssammlungseinheit umfasst:
eine erste Seite (111), die an einem Umfang an einer zweiten Seite (112) substantiell befestigt ist, so dass ein innerer Raum (121) zwischen den ersten und zweiten Seiten (111, 112) erzeugt wird, und
eine Öffnung (115) an der ersten Seite (111) von der Außenseite der Sammlungseinheit (101) zu dem inneren Raum (121), wobei die Öffnung (115) konfiguriert ist, um zu erlauben, dass Körperflüssigkeit während körperlicher Anstrengung innerhalb der Sammlungseinheit (101) durch die Öffnung fließt.

2. Erzeugnis nach Anspruch 1, wobei die Sammlungseinheit (101) umfasst:
eine absorbierende Schicht, die konfiguriert ist, um in substantiellem Kontakt mit dem Testindividuum während körperlicher Anstrengung zu stehen, und welche Schicht sich durch die Öffnung (115) der Sammlungseinheit (101) in den inneren Raum (121) erstreckt, wobei während einer Verwendung Körperflüssigkeit von dem Testindividuum durch die absorbierende Schicht und in die Sammlungseinheit (101) wandert.

3. Erzeugnis nach Anspruch 1, wobei die Körperflüssigkeit Schweiß ist.

4. Erzeugnis nach Anspruch 1, umfassend eine Tabelle, welche einen Schätzwert einer Menge von während körperlicher Aktivität sekretiertem Schweiß bereitstellt, und
umfassend Dosierungsinformation in Bezug auf die verzehrbare Zusammensetzung basierend auf dem Testergebnis für ein Ergänzen von Salz, welches ausreicht, um Muskelhrampf substantiell zu begrenzen.

5. Erzeugnis nach Anspruch 1 oder Anspruch 4, welches des Weiteren ein Testdiagramm (305) umfasst, welches konfiguriert ist, um mit der Testeinrichtung (204) nach Exposition gegenüber der Körperflüssigkeit verglichen zu werden, um das Testergebnis eines abgeschätzten Ausmaßes an Depletion von Salz oder Ionen bereitzustellen.

6. Erzeugnis von Anspruch 5, wobei das Testdiagramm (305) mindestens einen Abschnitt der Dosierungsinformation umfasst.

7. Erzeugnis von Anspruch 5, wobei das mindestens eine Salz einen Elektrolyt, ausgewählt aus der Gruppe bestehend aus: Natrium, Kalium, Magnesium, Calcium, Chlorid und Kombinationen davon, umfasst oder
wobei die verzehrbare Zusammensetzung aus der Gruppe bestehend aus: einem Pulver, einem Gel, einem Feststoff, einer Flüssigkeit und Kombinationen davon ausgewählt ist.

8. Erzeugnis von Anspruch 5, wobei die Testeinrichtung (204) konfiguriert ist, um die Konzentration von Bestandteilen, ausgewählt aus der Gruppe bestehend aus: Salz, Natrium, Chlorid und Kombinationen davon, zu messen, oder
wobei die Testeinrichtung (204) konfiguriert ist, um die Konzentration von Bestandteilen, ausgewählt aus der Gruppe bestehend aus: Proteinen, Harnstoff, Ketonen, Lactat, Magnesium, Kalium und Kombinationen davon, zu messen, oder
wobei die Testeinrichtung (204) konfiguriert ist, um Parameter, ausgewählt aus der Gruppe bestehend aus: relativer Dichte, pH und Kombinationen davon, zu messen, oder
wobei das Erzeugnis des Weiteren mindestens ein Getränk, auf welches auf einer Dosierungsinformation verwiesen wird, umfasst, oder
wobei das Erzeugnis des Weiteren ein Rehydratationsgetränkekonzentrat und Dosierungsinformation umfasst, wobei die Dosierungsinformation Information zum Herstellen eines Rehydratationsgetränks ausgehend von dem Rehydratationsgetränkekonzentrat umfasst.

9. Erzeugnis nach Anspruch 1 oder 2, wobei die Testeinrichtung konfiguriert ist, um in einem kolorimetrischen Test eingesetzt zu werden.

10. Verwendung eines Erzeugnisses nach einem der vorangegangenen Ansprüche zum Verringern von Muskelkrämpfen während körperlicher Aktivität.

## Revendications

1. Produit visant à réduire ou limiter les crampes musculaires, comprenant :
un emballage (450) comprenant une composition consommable ayant au moins un sel ;
un testeur (204) comprenant une partie de test (233) configurée pour être exposé à un fluide corporel à utiliser dans l'obtention d'un résultat de test indicatif d'un degré estimé d'appauvrissement en sel, ou ionique, sur un sujet de test ; et
une unité de collecte de fluide corporel (101) configurée pour être montée de manière dermique sur la peau d'un sujet de test afin de collecter un fluide corporel pendant l'exercice physique, dans lequel l'unité de collecte de fluide corporel comprend :
un premier côté (111) sensiblement fixé à un second côté (112) sur un périmètre, de façon à créer un espace interne (121) entre les premier et second côtés (111, 112) ; et
une ouverture (115) sur le premier côté (111) de l'extérieur de l'unité de collecte (101) vers l'espace interne (121), dans lequel l'ouverture (115) est configurée pour permettre au fluide corporel de s'écouler dans l'unité de collecte (101) à travers l'ouverture pendant l'exercice physique.

2. Produit selon la revendication 1, dans lequel l'unité de collecte (101) comprend :
une couche absorbante configurée pour être en contact substantiel avec le sujet de test pendant l'exercice physique et ladite couche s'étend à travers l'ouverture (115) de l'unité de collecte (101) dans l'espace interne (121), dans lequel, pendant l'utilisation, le fluide corporel se déplace depuis le sujet de test à travers la couche absorbante et dans l'unité de collecte (101).

3. Produit selon la revendication 1, dans lequel le fluide corporel est la transpiration.

4. Produit selon la revendication 1, comprenant un tableau fournissant une estimation de la quantité de sueur secrétée pendant l'activité physique, et
comprenant des informations de dosage concernant la composition consommable sur la base du résultat de test, afin de permettre un ravitaillement en sel suffisant pour limiter sensiblement les crampes musculaires.

5. Produit selon la revendication 1 ou la revendication 4 comprenant en outre un graphique de test (305) configuré pour être comparé au testeur (204) après exposition au fluide corporel, afin de fournir le résultat de test d'un degré estimé de l'appauvrissement en sel ou ionique.

6. Produit selon la revendication 5, dans lequel le graphique de test (305) comprend au moins une partie des informations de dosage.

7. Produit selon la revendication 5, dans lequel ledit au moins un sel comprend un électrolyte sélectionné dans le groupe constitué de : sodium, potassium, magnésium, calcium, chlorure et leurs combinaisons, ou
dans lequel la composition consommable est choisie dans le groupe constitué de : une poudre, un gel, un solide, un liquide et leurs combinaisons.

8. Produit selon la revendication 5, dans lequel le testeur (204) est configuré pour mesurer la concentration de composants choisis dans le groupe constitué de : sel, sodium, chlorure et leurs combinaisons, ou
dans lequel le testeur (204) est configuré pour mesurer la concentration de composants choisis dans le groupe constitué de : protéines, urée, cétones, lactate, magnésium, potassium et leurs combinaisons, ou
dans lequel le testeur (204) est configuré pour mesurer des paramètres choisis dans le groupe constitué de : gravité spécifique, pH, et leurs combinaisons, ou
dans lequel le produit comprend en outre au moins une boisson référencée sur les informations de dosage, ou
dans lequel le produit comprend en outre un concentré de boisson de réhydratation et des informations de dosage, les informations de dosage comprenant des informations sur la préparation d'une boisson de réhydratation à partir du concentré de boisson de réhydratation.

9. Produit selon la revendication 1 ou 2, dans lequel le testeur est configuré pour être utilisé dans un test colorimétrique.

10. Utilisation d'un produit selon l'une quelconque des revendications précédentes, afin de réduire les crampes musculaires pendant l'activité physique.
